(19) **Europäisches Patentamt / European Patent Office / Office européen des brevets**

(11) **EP 2 292 141 B1**

(12) **FASCICULE DE BREVET EUROPEEN**

(45) Date de publication et mention
de la délivrance du brevet:
**17.06.2015 Bulletin 2015/25**

(51) Int Cl.:
**A61B 5/024** *(2006.01)* **A61B 5/11** *(2006.01)*

(21) Numéro de dépôt: **09169391.1**

(22) Date de dépôt: **03.09.2009**

(54) **Procédé et dispositif de mesure du pouls au moyen d'ondes lumineuses à deux longueurs d'onde**

Verfahren und Vorrichtung zum Pulsmessen mit Hilfe von Lichtwellen mit zwei Wellenlängen

Method and device for taking a patient's pulse using light waves with two wavelengths

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(43) Date de publication de la demande:
**09.03.2011 Bulletin 2011/10**

(73) Titulaire: **The Swatch Group Research and Development Ltd**
**2074 Marin (CH)**

(72) Inventeur: **Jornod, M. Alain**
**2000 Neuchâtel (CH)**

(74) Mandataire: **Ravenel, Thierry Gérard Louis et al**
**ICB**
**Ingénieurs Conseils en Brevets SA**
**Faubourg de l'Hôpital 3**
**2001 Neuchâtel (CH)**

(56) Documents cités:
**EP-A1- 0 941 694     WO-A1-91/18550**
**WO-A2-96/12435     US-A- 5 595 176**

**Description**

L'invention concerne un procédé de mesure du pouls. Ce procédé permet de déterminer la pulsation cardiaque d'une personne sur la base d'un signal utile extrait au moyen du dispositif de mesure du pouls porté par la personne. Pour ce faire, le dispositif de mesure du pouls utilise notamment des ondes lumineuses à deux longueurs d'onde différentes.

L'invention concerne également un dispositif de mesure du pouls au moyen d'ondes lumineuses à deux longueurs d'onde différentes pour la mise en oeuvre du procédé.

Pour la mesure optique de la modulation du volume des vaisseaux sanguins définissant la pulsation cardiaque, il doit être tenu compte du fait que l'hémoglobine absorbe plus la lumière que les autres tissus à certaines longueurs d'onde. Pour ce faire, il est connu d'utiliser deux faisceaux de lumière à longueur d'onde différente pour la mesure notamment de la pulsation cardiaque.

A ce titre, on peut citer le brevet US 4,770,179, qui décrit un oxymètre. Cet oxymètre utilise une première diode LED rouge et une seconde diode LED infrarouge pour irradier les vaisseaux sanguins d'une personne, et un photorécepteur, tel qu'une photodiode pour capter la lumière réfléchie. Avec cet oxymètre, il est observé une source d'erreur importante du signal optique par la modulation dudit signal lors de mouvements de la personne. Cette modulation peut être de quelques ordres de grandeur supérieure à la modulation de l'absorption optique par le sang, ce qui constitue un inconvénient d'un tel oxymètre. De ce fait, il ne permet pas de tenir compte des mouvements de la personne pour déterminer précisément la modulation du volume des vaisseaux sanguins, c'est-à-dire la pulsation cardiaque.

Dans un même ordre d'idée, on peut citer le brevet FR 2 511 594, qui décrit un dispositif de contrôle du pouls. Ce dispositif comprend deux diodes LED infrarouges, et un photo-détecteur pour la mesure de la pulsation cardiaque d'un sportif en mouvement par exemple. Cependant, il n'est pas prévu de différencier précisément la mesure du pouls entre une personne en mouvement et une personne dans une position statique. Il n'est également pas tenu compte du fait que l'absorption par le sang est plus sélective en longueur d'onde que la modulation du signal par les mouvements de la personne pour permettre d'effectuer la mesure du pouls. De plus, rien n'est prévu pour supprimer également l'effet de la lumière ambiante qui est une autre source d'erreur pour la mesure du pouls.

Une solution au problème de la mesure du pouls lié au mouvement de la personne, est proposée dans la demande de brevet US 2005/0075553. Cette demande de brevet décrit un procédé et un appareil portable, tel qu'une montre-bracelet pour la gestion d'une information biologique, telle que la pulsation cardiaque et des fonctions du système nerveux. La montre-bracelet comprend un dispositif capteur ayant une diode LED de couleur verte, une diode LED infrarouge, et une photodiode pour capter la lumière réfléchie par la peau et les vaisseaux sanguins et qui provient des diodes LED enclenchées alternativement, et une unité à microcontrôleur reliée au dispositif capteur pour le commander. Les ondes lumineuses vertes permettent d'obtenir un signal de détection de la pulsation et du mouvement, alors que les ondes lumineuses infrarouges permettent d'obtenir essentiellement un signal du mouvement de la personne portant la montre.

L'unité à microcontrôleur reçoit les signaux numériques d'un convertisseur analogique/numérique du dispositif capteur. Cette unité effectue une transformée rapide de Fourier (FFT) des signaux numériques reçus des ondes lumineuses vertes et infrarouges. Après standardisation des spectres de Fourier, le spectre des ondes lumineuses infrarouges est soustrait du spectre des ondes lumineuses vertes de manière à pouvoir retirer l'effet du mouvement et calculer le pouls de la personne. Cependant, l'intensité des ondes lumineuses doit être bien adaptée pour permettre de retirer complètement l'effet du mouvement après l'opération de soustraction des spectres de Fourier des ondes lumineuses, ce qui est un inconvénient. De plus, une forte consommation électrique est constatée pour effectuer la mesure du pouls, ce qui constitue un autre inconvénient.

Selon un même principe que le précédent document, la demande de brevet EP 0 941 694 décrit un instrument de mesure du pouls, tel qu'une montre-bracelet. L'instrument comprend une première diode LED pour générer des ondes lumineuses rouges et une seconde diode LED pour générer des ondes lumineuses vertes. Il comprend encore deux photo-détecteurs pour capter les ondes lumineuses rouges et vertes réfléchies sur les tissus humains et dans les vaisseaux sanguins. Une unité de traitement permet d'appliquer un procédé FFT aux signaux captés pour le calcul du pouls. Cependant il doit également être prévu une normalisation des distances entre les photo-détecteurs et des intensités des ondes lumineuses générées et captées, ce qui constitue un inconvénient.

L'invention a donc pour but de pallier aux inconvénients de l'état de la technique susmentionné en fournissant un procédé de détermination de la pulsation cardiaque d'une personne au moyen d'un dispositif de mesure du pouls, qui permet de facilement déterminer le pouls sans être dépendant de l'intensité des ondes lumineuses générées et captées.

A cet effet, l'invention concerne un procédé de détermination de la pulsation cardiaque d'une personne au moyen d'un dispositif de mesure du pouls, qui comprend les caractéristiques définies dans la revendication indépendante 1.

Des étapes particulières du procédé de détermination de la pulsation cardiaque sont définies dans les revendications dépendantes 2 à 7.

Un avantage du procédé selon l'invention réside dans le fait qu'il est calculé une fonction spectrale de cohérence appliquée aux signaux de mesure fournis par le détecteur. La fonction de cohérence permet de pouvoir discriminer efficacement les contributions de la pulsation cardiaque et du mouvement de la personne. De cette fonction de cohérence, il est ainsi possible de déterminer un spectre de puissance moyen non cohérent duquel il est facile d'extraire le signal utile de pulsation. La différence d'intensité lumineuse captée par le détecteur n'a ainsi plus besoin d'être réglée précisément.

Un autre avantage du procédé selon l'invention réside dans le fait qu'il est également possible d'extraire un signal utile du mouvement de la personne sur la base d'un spectre de puissance moyen cohérent. La fréquence du mouvement de la personne peut ainsi être déterminée grâce à ce spectre de puissance moyen cohérent.

A cet effet, l'invention concerne également un dispositif de mesure du pouls convenant à la mise en oeuvre du procédé de détermination de la pulsation cardiaque d'une personne, qui comprend les caractéristiques définies dans la revendication indépendante 8.

Des formes d'exécution particulières du dispositif de mesure du pouls sont définies dans les revendications dépendantes 9 à 11.

A cet effet, l'invention concerne également un instrument électronique portable, tel qu'une montre-bracelet équipé d'un dispositif de mesure du pouls, qui comprend les caractéristiques de la revendication indépendante 12.

Les buts, avantages et caractéristiques du procédé de détermination de la pulsation cardiaque d'une personne et du dispositif pour sa mise en oeuvre apparaîtront mieux dans la description suivante d'au moins un mode de réalisation de l'invention en liaison avec les dessins dans lesquels :

la figure 1 représente un schéma bloc des différents composants du dispositif de mesure du pouls pour la mise en oeuvre du procédé selon l'invention,

la figure 2 représente un graphique temporel des signaux captés aux deux longueurs d'onde par le photo-détecteur du dispositif de mesure du pouls selon l'invention sans mouvement du porteur du dispositif,

la figure 3 représente un graphique temporel des signaux captés aux deux longueurs d'onde par le photo-détecteur du dispositif de mesure du pouls selon l'invention en présence de mouvement du porteur du dispositif,

la figure 4 représente un graphique des spectres de Fourier des signaux captés aux deux longueurs d'onde par le photo-détecteur du dispositif de mesure du pouls selon l'invention en présence de mouvement du porteur du dispositif,

la figure 5 représente un graphique du spectre de puissance de Fourier cohérent en présence de mouvement des signaux captés par le photo-détecteur du dispositif de mesure du pouls selon l'invention,

la figure 6 représente un graphique du spectre de puissance de Fourier non cohérent en présence de mouvement des signaux captés par le photo-détecteur du dispositif de mesure du pouls selon l'invention, et

la figure 7 représente des graphiques temporels des impulsions lumineuses générées par les sources de lumière et les mesures effectuées par le photo-détecteur et le convertisseur analogique-numérique du dispositif de mesure du pouls selon l'invention.

Dans la description suivante, tous les éléments du dispositif de mesure du pouls qui sont bien connus de l'homme du métier dans ce domaine technique ne seront relatés que de manière simplifiée, notamment en ce qui concerne l'unité à microcontrôleur pour le traitement des signaux numériques en liaison aux ondes lumineuses détectées. Le dispositif de mesure du pouls peut être intégré dans un instrument électronique portable pouvant être mis en contact avec la peau du porteur, tel qu'une montre-bracelet ou un téléphone portable ou un badge ou un autre instrument.

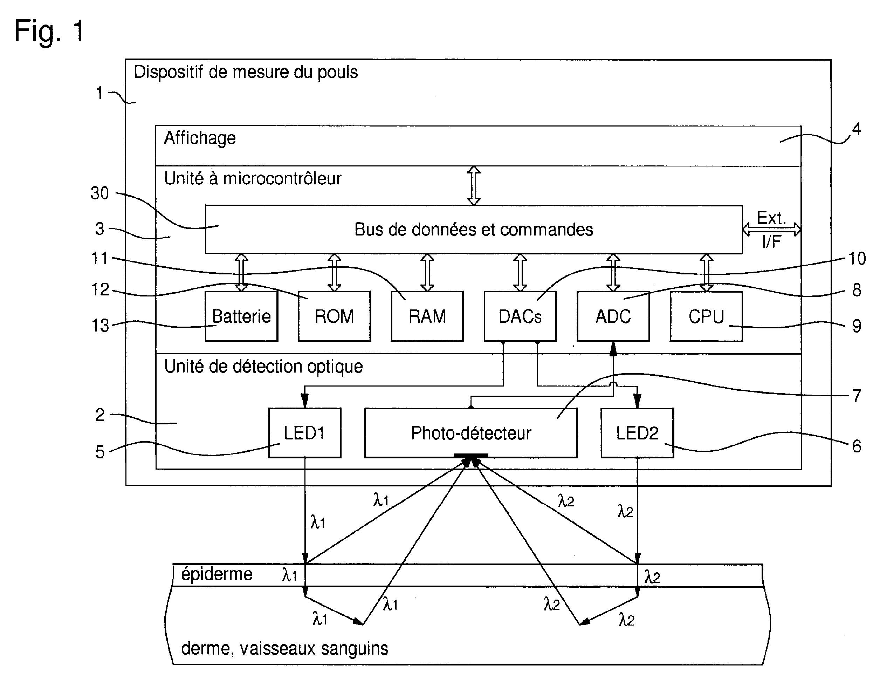

A la figure 1, il est représenté un dispositif de mesure du pouls 1. Ce dispositif comprend une unité de détection optique 2, qui est composée généralement de deux sources de lumière, telle que deux diodes électroluminescentes LED 5 et 6, et d'un photo-détecteur 7, qui comprend au moins une photodiode. Les diodes LED émettent chacune des ondes lumineuses ayant une longueur d'onde différente. Une première diode LED 5 produit des ondes lumineuses à une première longueur d'onde $\lambda_1$ située de préférence dans le domaine du vert, alors qu'une seconde diode LED 6 produit des ondes lumineuses à une seconde longueur d'onde $\lambda_2$ située de préférence dans le domaine du rouge ou infrarouge.

Le dispositif de mesure du pouls 1 comprend encore une unité à microcontrôleur 3 et une cellule d'affichage 4. Cette cellule d'affichage est reliée à l'unité à microcontrôleur 3 par l'intermédiaire d'un bus de données et commandes 30 de l'unité, qui relie également différents composants de l'unité à microcontrôleur 3. La cellule d'affichage 4 peut être un dispositif à cristaux liquides par exemple ou d'un autre type pour pouvoir afficher sur requête de l'utilisateur les informations relatives à la mesure du pouls. La cellule d'affichage commandée par l'unité à microcontrôleur, peut afficher les informations de la mesure du pouls en continu pendant la mesure du pouls ou après avoir été mémorisées dans les moyens de mémorisation à tout instant après la mesure du pouls. Plusieurs valeurs du pouls peuvent ainsi être mémorisées et affichées sur requête sur la cellule d'affichage.

L'unité à microcontrôleur est composée tout d'abord d'une source d'alimentation définie comme une batterie 13 pour l'alimentation de tous les composants du dispositif de mesure du pouls. Cette unité à microcontrôleur comprend encore un convertisseur numérique-analogique 10 pour fournir des signaux d'enclenchement respectivement à chaque diode LED 5 et 6 suite à une commande numérique fournie par une unité de traitement 9, tel qu'un processeur. L'unité à microcontrôleur comprend également un convertisseur analogique-numérique 8 pour recevoir les signaux de mesure captés par le photo-détecteur 7 et les convertir numériquement pour des opérations de traitement dans l'unité de traitement 9. L'unité à microcontrôleur comprend encore des moyens de mémorisation définis par exemple comme une mémoire du type ROM 12 et une mémoire du type RAM 11. La mémoire ROM 12 comprend toutes les instructions de mise en fonction de l'unité à microcontrôleur et des paramètres de configuration spécifiques. La mémoire du type RAM 11 est destinée à stocker, notamment pendant la mise en fonction du dispositif de mesure du pouls, des paramètres tirés des signaux numérisés fournis par le photo-détecteur 7 et traités dans l'unité de traitement 9.

L'unité à microcontrôleur 3 peut comprendre encore un étage oscillateur non représenté, qui peut être dépendant d'un résonateur à quartz non représenté. Dans le cas d'une utilisation dans une montre-bracelet, ladite unité à microcontrôleur peut comprendre une partie de l'étage oscillateur, qui est destiné à la base de temps de ladite montre. Dans ce cas, cet étage oscillateur est muni de plusieurs diviseurs pour fournir des signaux de cadencement de toutes les opérations traitées par l'unité de traitement 9 de l'unité à microcontrôleur 3.

En fonctionnement normal du dispositif de mesure du pouls 1 pendant une période de mesure déterminée, les diodes LED 5 et 6 sont enclenchées alternativement et de manière discontinue dans le temps sous la commande de l'unité de traitement 9. La première diode LED 5, qui fournit des ondes lumineuses à la première longueur d'onde $\lambda_1$, est enclenchée successivement dans le temps pendant des laps de temps déterminés. La seconde diode LED 6, qui fournit des ondes lumineuses à la seconde longueur d'onde $\lambda_2$, est enclenchée successivement dans le temps pendant des laps de temps déterminés, qui correspondent généralement aux laps de temps d'enclenchement de la première diode LED. Quand une des diodes LED est enclenchée, l'autre diode LED est généralement éteinte, et inversement tout en laissant des intervalles temporels où les deux diodes LED sont éteintes.

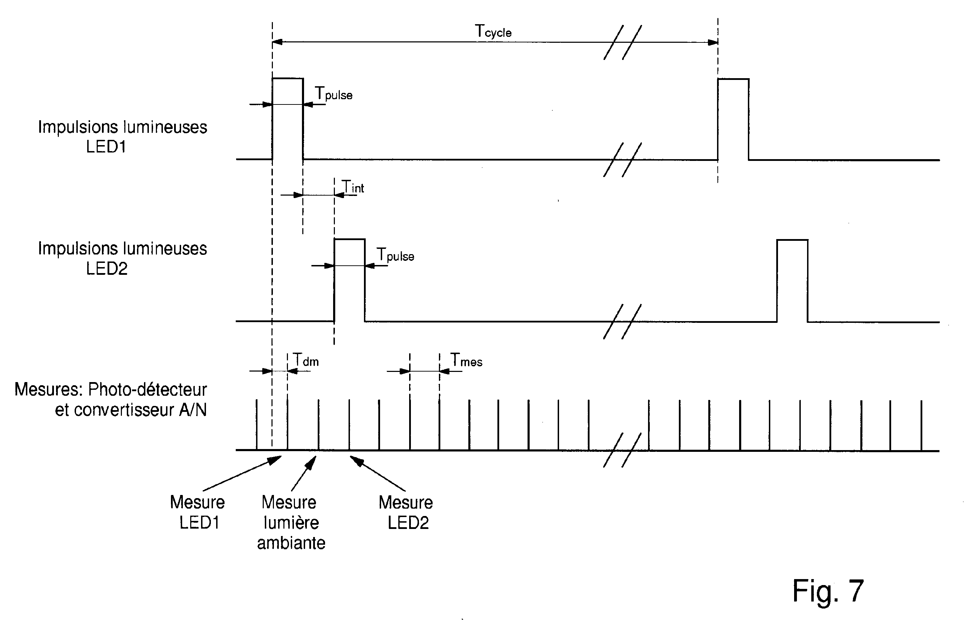

A titre d'exemple non limitatif, des graphiques temporels des ondes lumineuses générées par les sources de lumière et les signaux de mesure par l'intermédiaire du photo-détecteur et du convertisseur analogique-numérique sont représentés à la figure 7. Les diodes LED peuvent être alternativement enclenchées et de manière discontinue dans le temps. La première diode LED1 est tout d'abord enclenchée pendant un laps de temps déterminé $T_{pulse}$, qui peut être dans ce cas de l'ordre de 20 $\mu$s. La seconde diode LED2 est enclenchée un intervalle de temps de pause $T_{int}$ par exemple de l'ordre de 20 $\mu$s après la mise au repos de la première diode LED1. Le laps de temps d'enclenchement de la seconde diode LED2 est similaire à celui de la première diode LED1. Les impulsions lumineuses de chaque diode LED interviennent après chaque cycle $T_{cycle}$, qui peut être de l'ordre de 1 ms. Des mesures des ondes lumineuses captées de la première diode LED1, de la lumière ambiante et de la seconde diode LED2 sont effectuées à des périodes de mesure déterminées $T_{mes}$, qui peuvent être de l'ordre de 20 $\mu$s, mais en décalage $T_{dm}$ de 10 $\mu$s par rapport à chaque enclenchement des diodes LED.

Il est à noter qu'en lieu et place d'une modulation temporelle représentée notamment à la figure 7, il aurait pu être prévu d'effectuer une modulation en fréquence. Dans ce cas, les signaux de la première diode LED1 sont émis et détectés à une fréquence de l'ordre de 600 Hz, alors que ceux de la seconde diode LED2 sont émis et détectés à une fréquence de l'ordre de 900 Hz. Avec cela, il est effectué une modulation et démodulation fréquentielle.

Pour le procédé de détermination de la pulsation cardiaque, le dispositif de mesure du pouls 1 peut être mis en fonction par une commande externe, telle qu'au moyen d'un bouton d'un instrument électronique portable dans lequel il est placé. Le dispositif de mesure du pouls peut également être mis en fonction par programmation de manière automatique. Dans ces conditions, il peut être requis qu'une mesure du pouls, ainsi qu'un calcul de la fréquence du mouvement de la personne interviennent dans des périodes temporelles programmées suffisamment espacées l'une de l'autre.

La première longueur d'onde $\lambda_1$, qui est typiquement dans le vert, est choisie pour correspondre à une plus grande absorption optique par l'hémoglobine que la deuxième longueur d'onde $\lambda_2$, qui est typiquement dans le rouge. Les signaux de mesure provenant du photo-détecteur 7, qui a capté les ondes lumineuses réfléchies et/ou absorbées en partie par l'hémoglobine, sont enregistrés. Cet enregistrement, notamment dans la mémoire RAM 11, peut être effectué directement après numérisation des signaux de mesure par le convertisseur analogique-numérique 8. Cet enregistrement s'opère de façon continue tout en tenant compte des intervalles temporels où les deux sources de lumière sont éteintes.

Dans les intervalles temporels de pause, il est notamment prévu que l'unité de traitement 9 tienne compte de la lumière ambiante captée par le photo-détecteur 7. Ceci permet de retirer l'effet de la lumière ambiante lors de la mesure du pouls. A cet effet, une valeur moyenne de la lumière détectée lorsque les sources de lumière 5 et 6 sont éteintes, c'est-à-dire la lumière ambiante détectée, peut être soustraite en continu dans le domaine temporel avant une transformation dans le domaine fréquentiel. Il peut être imaginé également que le spectre de la lumière ambiante est soustrait aux spectres des ondes lumineuses aux deux longueurs d'onde $\lambda_1$ et $\lambda_2$ dans le domaine fréquentiel de Fourier.

Il est à noter que des paramètres de configuration Ext peuvent être fournis de l'extérieur par l'intermédiaire du bus de données et commandes 30. Des informations I/F notamment de mesure peuvent encore être transmises par l'intermédiaire du bus de données et commandes 30 vers l'extérieur du dispositif de mesure du pouls 1.

Le procédé de détermination de la pulsation cardiaque de la présente invention peut utiliser la fonction spectrale de cohérence notamment dans l'unité de traitement 9, qui peut être un processeur. Ce procédé permet d'extraire un signal utile de pulsation au moyen du dispositif de mesure du pouls 1. Cette fonction est une mesure du degré de dépendance entre deux signaux A et B dans le domaine fréquentiel. Les deux signaux A et B peuvent être considérés comme les deux signaux fournis par le photo-détecteur. Ces signaux A et B correspondent respectivement aux ondes lumineuses captées à la première longueur d'onde $\lambda_1$ et aux ondes lumineuses captées à la seconde longueur d'onde $\lambda_2$. La fonction $\gamma$ est calculée suivant une moyenne temporelle de spectres de Fourier des signaux A et B. Cette fonction $\gamma$ prend des valeurs comprises entre 0, pour laquelle aucune relation n'existe entre les deux signaux, et 1, pour laquelle il y a une relation linéaire parfaite. Cette fonction $\gamma$ est définie par la fonction suivante :

$$\gamma^2 = \frac{\left|\overline{S_{AB}}\right| \cdot \left|\overline{S_{AB}}\right|}{\overline{S_{AA}} \cdot \overline{S_{BB}}}$$

où $\overline{S_{AA}} = \overline{FFT(A) \cdot FFT^*(A)}$ et $\overline{S_{BB}} = \overline{FFT(B) \cdot FFT^*(B)}$ sont respectivement les spectres de puissance des signaux A et B et $\overline{S_{AB}} = \overline{FFT(A) \cdot FFT^*(B)}$ est la moyenne des spectres de Fourrier croisés des signaux A et B.

La fonction spectrale de cohérence $\gamma$ est habituellement utilisée pour déterminer la puissance spectrale cohérente $S_{CO} = \gamma^2 \cdot S_{BB}$, qui représente la partie cohérente entre les deux signaux A et B, qui n'est pas affectée par la partie non cohérente, représentée essentiellement par le bruit. Cette fonction spectrale $\gamma$ peut aussi être utilisée pour calculer le rapport signal/bruit d'un système S/N= $\gamma^2 / (1-\gamma^2)$.

Pour le procédé de la présente invention et comme indiqué ci-devant, les deux signaux A et B sont considérés comme les réponses aux deux longueurs d'onde $\lambda_1$ et $\lambda_2$. Les contributions du mouvement sont présentes dans les signaux A et B. Elles représentent donc la partie cohérente des spectres de Fourier. La modulation des signaux optiques par la pulsation sanguine, qui est essentiellement contenue dans le signal A, représente quant à elle la partie du spectre du signal A non cohérente avec celle du signal B. La fonction, qui contient le plus d'information sur la pulsation est donc la puissance spectrale non cohérente donnée par la formule suivante :

$$S_{NC} = (1 - \gamma^2) \cdot \overline{S_{AA}}$$

C'est de cette fonction, que l'unité à microcontrôleur 3, notamment par l'intermédiaire du processeur, doit extraire du signal utile, la fréquence de pulsation déterminant le pouls de la personne.

Il aurait pu être également envisagé d'utiliser un procédé de détermination de la pulsation en présence de modulation liée à des mouvements, qui est basé sur des différences de réponse du tissu en fonction de la longueur d'onde de la lumière. Ce procédé de détermination peut utiliser un facteur de normalisation dans le domaine de Fourier entre les spectres des deux longueurs d'onde $\lambda_1$ et $\lambda_2$, comme dans l'état de la technique. Pour ce faire, le facteur de normalisation entre les spectres de $\lambda_1$ et $\lambda_2$ est calculé dans les fréquences supérieures du spectre de Fourier, là où le contenu spectral est dominé par la modulation du signal liée au mouvement. Le facteur de normalisation peut être une valeur RMS du spectre dans un domaine compris entre deux fréquences, qui sont typiquement 6 et 10 Hz.

Une fois la normalisation effectuée, le spectre de la seconde longueur d'onde $\lambda_2$ est soustrait de celui de la première longueur d'onde $\lambda_1$. La modulation des signaux optiques par les mouvements est semblable pour les deux longueurs d'onde. La partie principale du spectre due aux vibrations ou mouvements est donc éliminée par soustraction. Cette soustraction dégrade, mais d'une manière non déterminante, l'amplitude du signal modulé. Dans l'exemple où la modulation des signaux optiques par la pulsation sanguine est dix fois plus grande pour la première longueur d'onde $\lambda_1$ que pour la seconde longueur d'onde $\lambda_2$, la diminution du signal de pulsation après l'opération de soustraction n'est que de 10%.

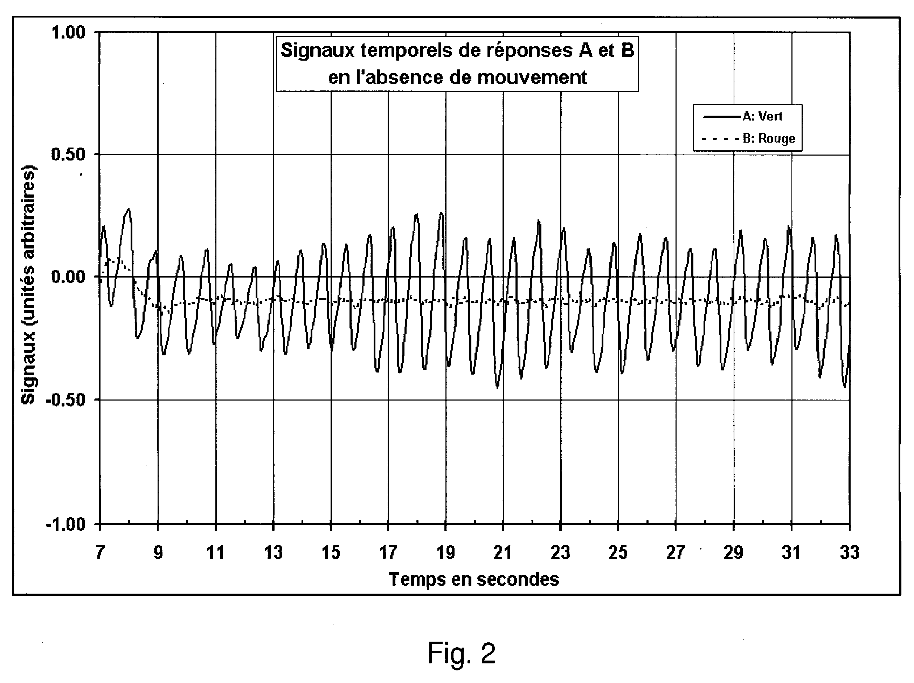

Pour mieux comprendre la forme des signaux captés dans le temps, ainsi que les spectres calculés par le dispositif de mesure du pouls, on peut se référer aux figures 2 à 6 décrites ci-après.

A la figure 2, il est tout d'abord représenté des signaux A et B dans le temps captés par le photo-détecteur provenant des ondes lumineuses émises par les deux diodes LED à la première longueur d'onde $\lambda_1$ (A) et à la seconde longueur d'onde $\lambda_2$ (B). On remarque sur ce graphique en gras les signaux captés par le photo-détecteur relatifs à la première longueur d'onde et en traits interrompus ceux captés par le photo-détecteur relatifs à la seconde longueur d'onde en l'absence de mouvement de la personne qui porte ledit dispositif.

Normalement, ces signaux sont décalés dans le temps l'un par rapport à l'autre étant donné l'enclenchement alternatif et discontinu des diodes LED utilisées pour cette mesure du pouls. Comme les signaux à la première longueur d'onde dans le vert sont sensibles à la pulsation, il n'est représenté qu'une vision de la pulsation par les ondes lumineuses à la première longueur d'onde $\lambda_1$, c'est-à-dire environ 3 cycles en 2 secondes (1.5 Hz), car peu de fluctuation est constatée pour les ondes lumineuses à la seconde longueur d'onde $\lambda_2$.

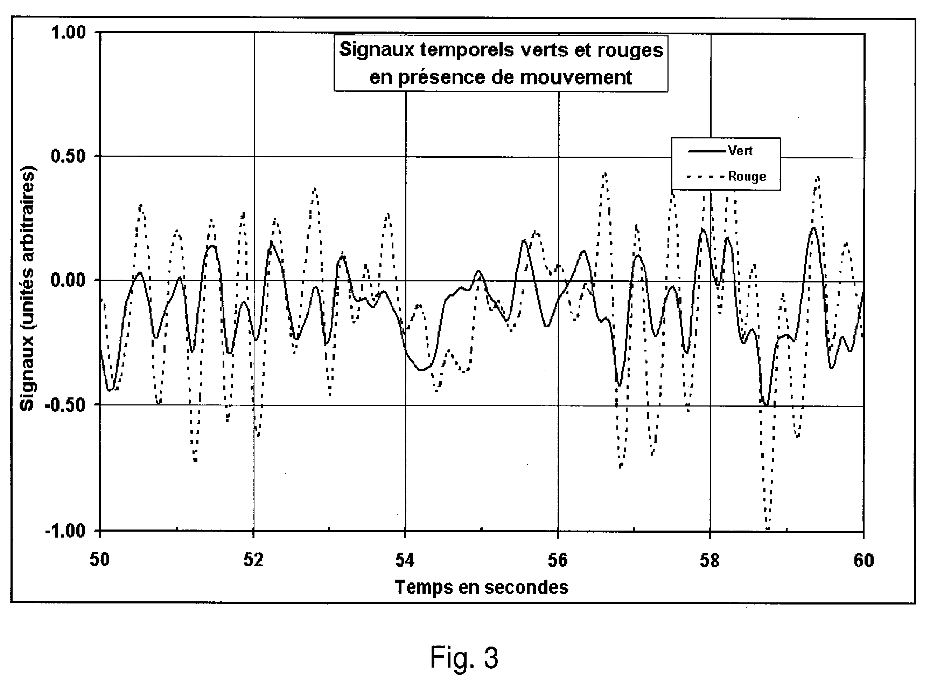

A la figure 3, il est représenté en présence de mouvement du porteur du dispositif, des signaux A et B dans le temps captés par le photo-détecteur provenant principalement des ondes lumineuses émises par les deux diodes LED. Ces ondes lumineuses sont réfléchies et/ou absorbées par l'hémoglobine avant d'être captées par le photo-détecteur. On remarque cette fois-ci que la pulsation est représentée beaucoup moins clairement sur lesdits signaux A et B fournis par le photo-détecteur.

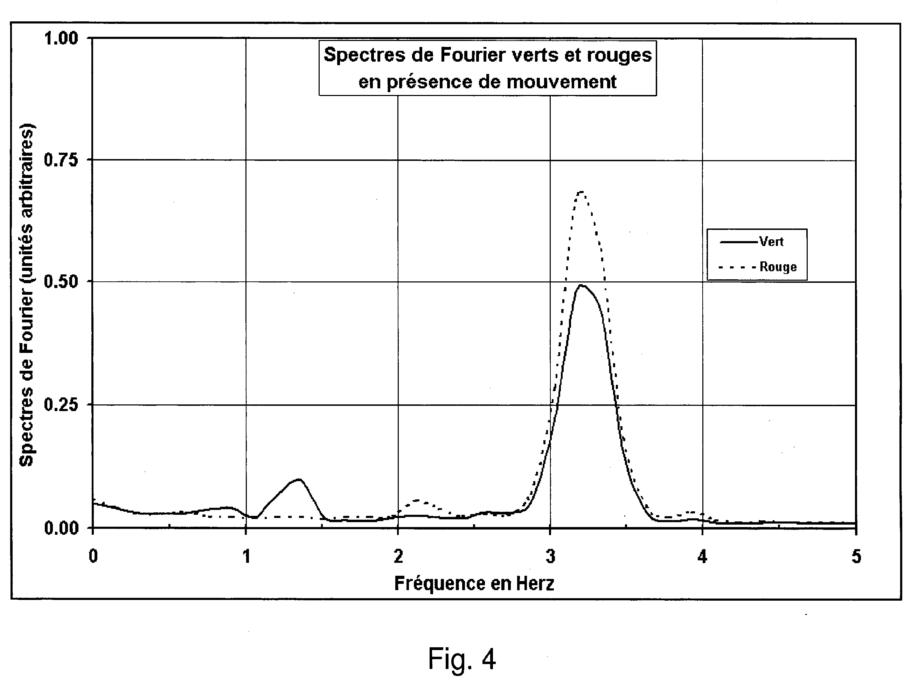

A la figure 4, il est représenté les spectres de Fourier des signaux A et B fournis par le photo-détecteur sur la base des ondes lumineuses aux deux longueurs d'onde captées. Les spectres de Fourier des signaux A et B sont obtenus en fonction des signaux A et B dans le domaine temporel, qui ont été enregistrés comme représentés à la figure 3. On remarque sur la figure 4, la contribution due au mouvement à environ 3.2 Hz des signaux A et B, alors que la pulsation se situe à environ 1.3 Hz et n'est visible que pour les signaux A à la première longueur d'onde.

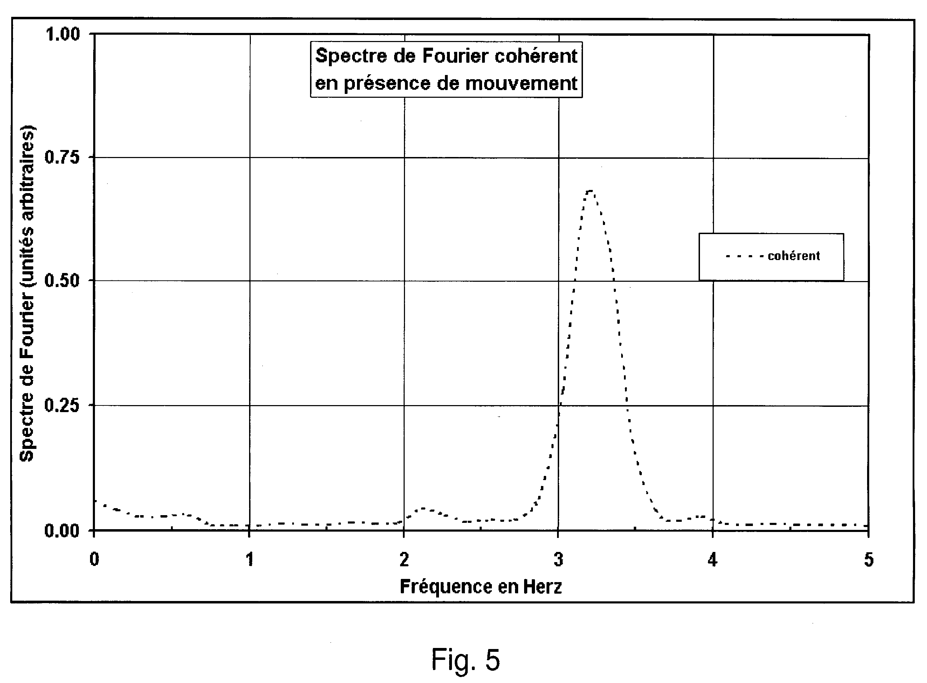

A la figure 5, il n'est représenté que le spectre de puissance de Fourier cohérent des signaux A et B fournis par le photo-détecteur sur la base des ondes lumineuses aux deux longueurs d'onde captées. Ce spectre de puissance cohérent est obtenu en fonction des signaux A et B dans le domaine temporel comme représentés à la figure 3.

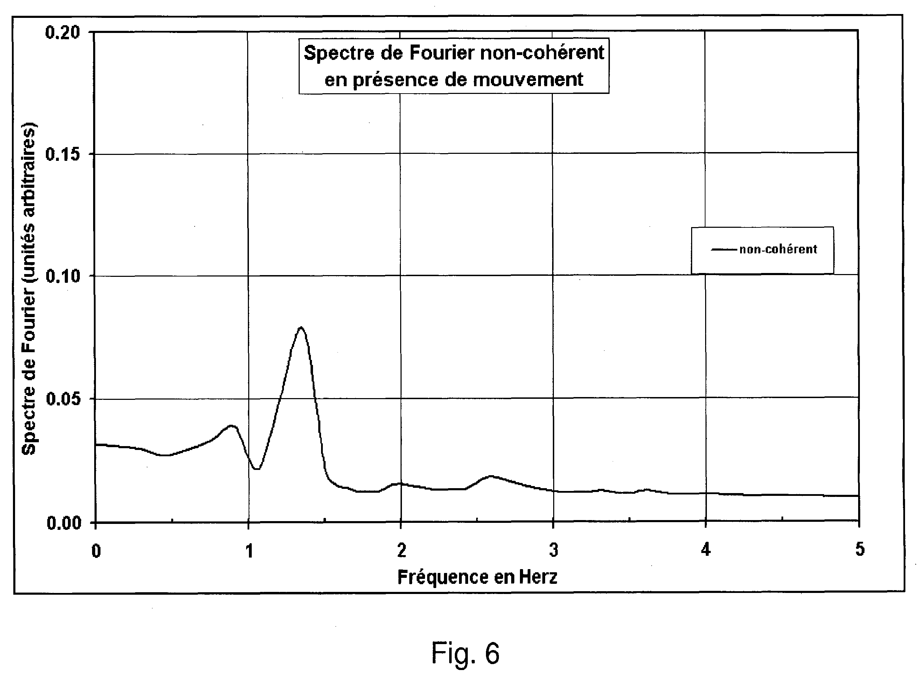

Finalement à la figure 6, il n'est représenté que le spectre de puissance de Fourier non cohérent des signaux A et B fournis par le photo-détecteur sur la base des ondes lumineuses aux deux longueurs d'onde captées. Ce spectre de puissance non cohérent est obtenu en fonction des signaux A et B dans le domaine temporel comme représentés à la figure 3.

Sur les figures 5 et 6, on constate que les contributions du mouvement et de la pulsation sont clairement discriminées. La composante du mouvement à environ 3.2 Hz n'apparaît clairement que sur le spectre cohérent de la figure 5, alors que la composante de pulsation à environ 1.3 Hz n'apparaît clairement que sur le spectre non cohérent de la figure 6. Dans ces conditions, il est facile à l'unité de traitement de l'unité à microcontrôleur d'extraire le signal utile de pulsation cardiaque directement du spectre de puissance non cohérent représenté à la figure 6. Bien entendu, ladite unité de traitement peut également être en mesure de déterminer la fréquence du mouvement de la personne portant ledit dispositif de mesure du pouls sur la base du spectre de puissance cohérent comme représenté à la figure 5.

A partir de la description qui vient d'être faite, plusieurs variantes du procédé et du dispositif de mesure du pouls peuvent être conçues par l'homme du métier sans sortir du cadre de l'invention définie par les revendications. Il peut être envisagé d'enclencher simultanément les deux sources de lumière et d'opérer une sélection de longueur d'onde au niveau du photo-détecteur. Le dispositif de mesure du pouls peut être intégré dans une montre-bracelet avec les sources de lumière et le photo-détecteur disposés du côté du fond du boîtier de la montre en contact direct avec la peau du porteur. L'émission des ondes lumineuses et la capture des ondes lumineuses réfléchies sont effectuées à travers une ou plusieurs ouvertures ou des portions transparentes du fond du boîtier.

## Revendications

1. Procédé de détermination de la pulsation cardiaque d'une personne au moyen d'un dispositif de mesure du pouls (1), qui comprend une première source de lumière (5) pour émettre des ondes lumineuses à une première longueur d'onde ($\lambda_1$), une seconde source de lumière (6) pour émettre des ondes lumineuses à une seconde longueur d'onde ($\lambda_2$), et un photo-détecteur (7) pour capter la lumière réfléchie par les tissus de la peau et les vaisseaux sanguins et fournir des signaux de mesure à une unité à microcontrôleur (3), les première et seconde longueurs d'onde étant différentes et déterminées pour que les ondes lumineuses à la première longueur d'onde soient sensibles à la pulsation et au mouvement d'une personne, et que les ondes lumineuses à la seconde longueur d'onde soient sensibles au mouvement d'une personne,

le procédé comprenant les étapes de :

   - générer des ondes lumineuses à la première longueur d'onde ($\lambda_1$) et à la seconde longueur d'onde ($\lambda_2$) au moyen des première (5) et seconde (6) sources lumineuses, et les transmettre en direction de la peau de la personne,
   - détecter les ondes lumineuses aux première et seconde longueurs d'onde, qui sont réfléchies par les tissus de la peau et les vaisseaux sanguins pour fournir des signaux de mesure électriques,

   **caractérisé en ce que** le procédé comprend additionnellement les étapes de

   - calculer au moins un spectre de puissance non cohérent sur la base d'une fonction spectrale de cohérence $\gamma$ qui tient compte de la dépendance entre les signaux de mesure relatifs à la première longueur d'onde et à la seconde longueur d'onde par la formule $S_{NC}=(1-\gamma^2)\cdot\overline{S_{AA}}$, où $S_M$ est le spectre de puissance moyen de Fourier des signaux de mesure relatifs à la première longueur d'onde des ondes lumineuses captées et où $\gamma^2$ est compris entre 0 et 1, et

- déterminer la pulsation cardiaque de la personne au moyen du spectre de puissance non cohérent.

2. Procédé de détermination de la pulsation cardiaque et du mouvement d'une personne au moyen du dispositif de mesure du pouls selon la revendication 1, **caractérisé en ce qu'**il est calculé au moins un spectre de puissance cohérent sur la base d'une fonction spectrale de cohérence *y,* par la formule $S_{CO}=\gamma^2 \cdot \overline{S_{BB}}$, où $\overline{S_{BB}}$ est le spectre de puissance moyen de Fourier des signaux de mesure relatifs à la seconde longueur d'onde ($\lambda_2$) des ondes lumineuses captées, et où $\gamma^2$ est compris entre 0 et 1, et **en ce qu'**il est déterminé la fréquence du mouvement de la personne au moyen du spectre de puissance cohérent.

3. Procédé de détermination de la pulsation cardiaque d'une personne selon la revendication 1, **caractérisé en ce que** les sources de lumière (5, 6) sont alternativement enclenchées pour la génération des ondes lumineuses aux deux longueurs d'onde.

4. Procédé de détermination de la pulsation cardiaque d'une personne selon la revendication 3, **caractérisé en ce que** les sources de lumière (5, 6) sont enclenchées alternativement et de manière discontinue dans le temps pendant des laps de temps déterminés, des intervalles temporels, pendant lesquels les sources de lumière sont éteintes, étant prévus entre chaque alternance d'enclenchement des sources de lumière.

5. Procédé de détermination de la pulsation cardiaque d'une personne selon la revendication 4, **caractérisé en ce qu'**une valeur moyenne de la lumière détectée par le photo-détecteur (7), lorsque les sources de lumière (5, 6) sont éteintes, est soustraite en continu dans le domaine temporel avant une transformation dans le domaine fréquentiel dans l'unité à microcontrôleur.

6. Procédé de détermination de la pulsation cardiaque d'une personne selon l'une des revendications 3 à 5, **caractérisé en ce que** le dispositif de mesure du pouls (1) est mis en fonction manuellement ou automatiquement pendant une période de mesure déterminée pour la fourniture de la mesure du pouls.

7. Procédé de détermination de la pulsation cardiaque d'une personne selon la revendication 6, **caractérisé en ce que** le dispositif de mesure du pouls (1) est mis en fonction automatiquement par période temporelle programmée de plusieurs ordres de grandeur plus grande que chaque période de mesure déterminée.

8. Dispositif de mesure du pouls (1) pour la mise en oeuvre du procédé selon l'une des revendications précédentes, le dispositif comprenant une unité de détection optique (2), qui comprend une première source de lumière (5) pour émettre des ondes lumineuses à une première longueur d'onde ($\lambda_1$), une seconde source de lumière (6) pour émettre des ondes lumineuses à une seconde longueur d'onde ($\lambda_2$), et un photo-détecteur (7) pour capter la lumière réfléchie par les tissus de la peau et les vaisseaux sanguins et fournir des signaux de mesure à une unité à microcontrôleur (3), **caractérisé en ce que** l'unité à microcontrôleur (3) comprend au moins une unité de traitement (9) susceptible de calculer au moins un spectre de puissance non cohérent sur la base d'une fonction spectrale de cohérence $\gamma$ qui tient compte de la dépendance entre les signaux de mesure fournis par le photo-détecteur et relatifs à la première longueur d'onde et à la seconde longueur d'onde afin de déterminer la pulsation cardiaque de la personne au moyen du spectre de puissance non cohérent.

9. Dispositif de mesure du pouls (1) selon la revendication 8, **caractérisé en ce que** l'unité à microcontrôleur (3) comprend des moyens de mémorisation (11, 12) dans lesquels sont enregistrés des instructions de mise en fonction de l'unité à microcontrôleur, des paramètres de configuration spécifique de ladite unité, des données relatives aux signaux de mesure numérisés fournis par le photo-détecteur et traités par l'unité de traitement, et **en ce que** l'unité de traitement (9) est susceptible de calculer au moins un spectre de puissance cohérent sur la base d'une fonction spectrale de cohérence $\gamma$ afin de déterminer la fréquence du mouvement de la personne au moyen du spectre de puissance cohérent.

10. Dispositif de mesure du pouls (1) selon la revendication 8, **caractérisé en ce qu'**il comprend une cellule d'affichage (4) commandée par l'unité à microcontrôleur (3), ladite cellule d'affichage étant capable d'afficher des informations relatives à la pulsation cardiaque et/ou au mouvement de la personne portant ledit dispositif.

11. Dispositif de mesure du pouls (1) selon la revendication 8, **caractérisé en ce que** l'unité à microcontrôleur (3) comprend un convertisseur analogique-numérique (8) pour convertir numériquement les signaux de mesure fournis par le photo-détecteur (7) afin d'être traités par l'unité de traitement (9), et au moins un convertisseur numérique-analogique (10) pour commander sur la base de signaux de commande de l'unité de traitement, alternativement l'enclenchement de chaque source de lumière (5, 6), chaque source de lumière étant une diode électroluminescente.

12. Instrument électronique portable, tel qu'une montre-bracelet, qui comprend un dispositif de mesure du pouls (1) selon l'une des revendications 8 à 11, **caractérisé en ce que** les première et seconde sources de lumière (5, 6) sont disposées dans un boîtier de l'instrument pour fournir des ondes lumineuses à travers un fond du boîtier, et **en ce que** le photodétecteur (7) est prévu pour capter des ondes lumineuses réfléchies à travers une ouverture ou portion transparente du fond du boîtier.

**Patentansprüche**

1. Verfahren zum Bestimmen des Herzschlags einer Person mittels einer Pulsmessvorrichtung (1), die eine erste Lichtquelle (5) zum Aussenden von Lichtwellen mit einer ersten Wellenlänge ($\lambda_1$), eine zweite Lichtquelle (6) zum Aussenden von Lichtwellen mit einer zweiten Wellenlänge ($\lambda_2$) und einen Photo-detektor (7) zum Empfangen des vom Gewebe der Haut und von den Blutgefäßen reflektierten Lichts und zum Liefern von Messsignalen an eine Mikrocontroller-Einheit (3) umfasst, wobei die erste und die zweite Wellenlänge voneinander verschieden sind und so bestimmt sind, dass die Lichtwellen mit der ersten Wellenlänge für den Pulsschlag und die Bewegung einer Person empfindlich sind und die Lichtwellen mit der zweiten Wellenlänge für die Bewegung einer Person empfindlich sind, wobei das Verfahren die folgenden Schritte umfasst:

- Erzeugen von Lichtwellen mit der ersten Wellenlänge ($\lambda_1$) und mit der zweiten Wellenlänge ($\lambda_2$) mittels der ersten Lichtquelle (5) bzw. der zweiten Lichtquelle (6) und Übertragen der Lichtwellen in Richtung der Haut der Person,
- Detektieren der Lichtwellen mit der ersten und der zweiten Wellen-länge, die vom Gewebe der Haut und den Blutgefäßen reflektiert werden, um elektrische Messsignale bereitzustellen,

**dadurch gekennzeichnet, dass** das Verfahren außerdem die folgenden Schritte umfasst:

- Berechnen wenigstens eines nicht kohärenten Leistungsspektrums anhand einer Spektralfunktion mit Kohärenz y, die die Abhängigkeit zwischen den Messsignalen in Bezug auf die erste Wellenlänge und die zweite Wellenlänge berücksichtigt, anhand der Formel $S_{NC} = (1-\gamma^2) \cdot \overline{S_{AA}}$, wobei $\overline{S_{AA}}$ das mittlere Fourier-Leistungsspektrum der Messsignale bezüglich der ersten Wellenlänge der empfangenen Lichtwellen ist und wobei $\gamma^2$ im Bereich von 0 bis 1 liegt, und
- Bestimmen des Herzimpulses der Person mittels des nicht kohärenten Leistungsspektrums.

2. Verfahren zum Bestimmen des Herzschlags und der Bewegung einer Person mittels der Pulsmessvorrichtung nach Anspruch 1, dadurch gekenn-zeichnet, dass wenigstens ein kohärentes Leistungsspektrum anhand einer Spektralfunktion mit Kohärenz $\gamma$ durch die Formel $S_{CO} = \gamma^2 \cdot \overline{S_{BB}}$ berechnet wird, wobei $\overline{S_{BB}}$ das mittlere Fourier-Leistungsspektrum der Messsignale in Bezug auf die zweite Wellenlänge ($\lambda_2$) der empfangenen Lichtwellen ist und wobei $\gamma^2$ im Bereich von 0 bis 1 liegt, und dass die Frequenz der Bewegung der Person mittels des kohärenten Leistungsspektrums bestimmt wird.

3. Verfahren zum Bestimmen des Herzschlags einer Person nach Anspruch 1, **dadurch gekennzeichnet, dass** die Lichtquellen (5, 6) abwechselnd eingeschaltet werden, um Lichtquellen mit den zwei Wellenlängen zu erzeugen.

4. Verfahren zum Bestimmen des Herzschlags einer Person nach Anspruch 3, **dadurch gekennzeichnet, dass** die Lichtquellen (5, 6) abwechselnd und zeitlich diskontinuierlich während bestimmter Zeitabschnitte eingeschaltet werden, wobei zwischen jedem Einschaltwechsel der Lichtquellen Zeitintervalle, in denen die Lichtquellen ausgeschaltet sind, vorgesehen sind.

5. Verfahren zum Bestimmen des Herzschlags einer Person nach Anspruch 4, **dadurch gekennzeichnet, dass** ein Mittelwert des durch den Photo-detektor (7) detektierten Lichts, wenn die Lichtquellen (5,6) ausgeschaltet sind, in dem Zeitbereich vor einer Transformation in den Frequenzbereich in der Mikro-controller-Einheit kontinuierlich subtrahiert wird.

6. Verfahren zum Bestimmen des Herzschlags einer Person nach einem der Ansprüche 3 bis 5, **dadurch gekennzeichnet, dass** die Pulsmessvorrichtung (1) manuell oder automatisch während einer Messperiode, die für die Bereitstellung der Pulsmessung bestimmt ist, in Betrieb versetzt wird.

7. Verfahren zum Bestimmen des Herzschlags einer Person nach Anspruch 6, **dadurch gekennzeichnet, dass** die Pulsmessvorrichtung (1) automatisch pro programmierter Zeitperiode, die um mehrere Größenordnungen größer ist als jede bestimmte Messperiode, in Betrieb versetzt wird.

8. Vorrichtung (1) zum Messen des Pulses durch Ausführen des Verfahrens nach einem der vorhergehenden Ansprüche, wobei die Vorrichtung eine optische Detektionseinheit (2) umfasst, die eine erste Lichtquelle (5) zum Aussenden von Lichtwellen mit einer ersten Wellenlänge ($\lambda_1$), eine zweite Lichtquelle (6) zum Aussenden von Lichtwellen mit einer zweiten Wellenlänge ($\lambda_2$) und einen Photodetektor (7) zum Empfangen des vom Gewebe der Haut und von den Blutgefäßen reflektierten Lichts und zum Liefern von Messsignalen an eine Mikrocontroller-Einheit (3) umfasst, **dadurch gekennzeichnet, dass** die Mikrocontroller-Einheit (3) wenigstens eine Verarbeitungseinheit (9) umfasst, die wenigstens ein nicht kohärentes Leistungsspektrum anhand einer Spektralfunktion mit Kohärenz $\gamma$ berechnen kann, die die Abhängigkeit zwischen den Messsignalen, die von dem Photodetektor geliefert werden und mit der ersten Wellenlänge und der zweiten Wellenlänge in Beziehung stehen, berücksichtigt, um den Herzschlag der Person mittels des nicht kohärenten Leistungsspektrums zu bestimmen.

9. Pulsmessvorrichtung (1) nach Anspruch 8, **dadurch gekennzeichnet, dass** die Mikrocontroller-Einheit (3) Speichermittel (11, 12) umfasst, in denen Befehle, die von der Mikrocontroller-Einheit ausgeführt werden, für die Einheit spezifische Konfigurationsparameter, digitale Daten bezüglich der Messsignale, die von dem Photodetektor geliefert und durch die Verarbeitungseinheit verarbeitet werden, aufgezeichnet sind, und dass die Verarbeitungseinheit (9) wenigstens ein kohärentes Leistungsspektrum anhand einer Spektralfunktion mit Kohärenz $\gamma$ berechnen kann, um die Frequenz der Bewegung der Person mittels des kohärenten Leistungsspektrums zu bestimmen.

10. Pulsmessvorrichtung (1) nach Anspruch 8, **dadurch gekennzeichnet, dass** sie eine Anzeigezelle (4) umfasst, die durch die Mikrocontroller-Einheit (3) gesteuert wird, wobei die Anzeigezelle Informationen bezüglich des Herzschlags und/oder der Bewegung der Person, die die Vorrichtung trägt, anzeigen kann.

11. Pulsmessvorrichtung (1) nach Anspruch 8, **dadurch gekennzeichnet, dass** die Mikrocontroller-Einheit (3) einen Analog/Digital-Umsetzer (8), um die von dem Photodetektor (7) gelieferten Messsignale digital umzusetzen, damit sie durch die Verarbeitungseinheit (9) verarbeitet werden, und wenigstens einen Digital/Analog-Umsetzer (10), um anhand der Befehlssignale der Verarbeitungseinheit abwechselnd das Einschalten jeder Lichtquelle (5, 6) zu steuern, umfasst, wobei jede Lichtquelle eine Elektrolumineszenzdiode ist.

12. Tragbares elektronisches Instrument wie etwa eine Armbanduhr, die eine Pulsmessvorrichtung (1) nach einem der Ansprüche 8 bis 11 umfasst, **dadurch gekennzeichnet, dass** die erste und die zweite Lichtquelle (5, 6) in einem Gehäuse des Instruments angeordnet sind, um Lichtwellen durch einen Boden des Gehäuses bereitzustellen, und dass der Photodetektor (7) vorgesehen ist, um Lichtwellen zu empfangen, die durch eine Öffnung oder einen lichtdurchlässigen Abschnitt des Bodens des Gehäuses reflektiert werden.

**Claims**

1. Method for determining the heartbeat of a person by means of a pulse measuring device (1), which comprises a first light source (5) for emitting light waves with a first wavelength ($\lambda_1$), a second light source (6) for emitting light waves with a second wavelength ($\lambda_2$), and a photodetector (7) for sensing the light reflected by the tissues of the skin and the blood vessels and for providing measurement signals to a microcontroller unit (3), the first and second wavelengths being different and determined so that the light waves with the first wavelength are sensitive to the pulse beat and to the movement of a person, and so that the light waves with the second wavelength are sensitive to movement of a person, the method comprising the steps of:

   - generating light waves with the first wavelength ($\lambda_1$) and the second wavelength ($\lambda_2$) by means of the first (5) and second (6) light sources, and transmitting them in the direction of the skin of the person,
   - detecting the light waves with the first and second wavelengths which are reflected by the tissues of the skin and the blood vessels in order to provide electrical measurement signals,

   **characterised in that** the method further comprises the steps of:

   - calculating at least one non-coherent power spectrum on the basis of a spectral coherence function $\gamma$ which takes into account the dependence between the measurement signals relating to the first wavelength and to the second wavelength by the formula $S_{NC} = (1-\gamma^2) \cdot \overline{S_{AA}}$, where $\overline{S_{AA}}$ is the average Fourier power spectrum of the measurement signals relating to the first wavelength of the sensed light waves and where $\gamma^2$ is between 0 and 1, and
   - determining the heartbeat of the person by means of the non-coherent power spectrum.

2. Method for determining the heartbeat and movement of a person by means of the pulse measuring device according to claim 1, **characterised in that** there is calculated at least one coherent power spectrum on the basis of a spectral coherence function $\gamma$, by the formula $SCO = \gamma^2 \cdot \overline{S_{BB}}$, where $\overline{S_{BB}}$ is the average Fourier power spectrum of the measurement signals relating to the second wavelength ($\lambda_2$) of the sensed light waves and where $\gamma^2$ is between 0 and 1, and **in that** there is determined the frequency of the movement of the person by means of the coherent power spectrum.

3. Method for determining the heartbeat of a person according to claim 1, **characterised in that** the light sources (5, 6) are switched on alternately for generation of the light waves with two wavelengths.

4. Method for determining the heartbeat of a person according to claim 3, **characterised in that** the light sources (5, 6) are switched on alternately and discontinuously in time during determined periods of time, temporal intervals, during which the light sources are switched off, being provided between each alternate activation of the light sources.

5. Method for determining the heartbeat of a person according to claim 4, **characterised in that** an average value of the light detected by the photodetector (7), when the light sources (5, 6) are switched off, is subtracted continuously in the temporal range before a transformation into the frequency range in the microcontroller unit.

6. Method for determining the heartbeat of a person according to one of the claims 3 to 5, **characterised in that** the pulse measuring device (1) is set in operation manually or automatically during a determined measuring period for provision of the pulse measurement.

7. Method for determining the heartbeat of a person according to claim 6, **characterised in that** the pulse measuring device (1) is set in operation automatically by a programmed temporal period of several orders of magnitude greater than each determined measuring period.

8. Pulse measuring device (1) for implementing the method according to one of the preceding claims, the device comprising an optical detection unit (2) which comprises a first light source (5) for emitting light waves with a first wavelength ($\lambda_1$), a second light source (6) for emitting light waves with a second wavelength ($\lambda_2$), and a photodetector (7) for sensing the light reflected by the tissues of the skin and the blood vessels and for providing measurement signals to a microcontroller unit (3), **characterised in that** the microcontroller unit (3) comprises at least one processing unit (9) which is able to calculate at least one non-coherent power spectrum on the basis of a spectral coherence function $\gamma$ which takes into account the dependence between the measurement signals provided by the photodetector and relating to the first wavelength and to the second wavelength in order to determine the heartbeat of the person by means of the non-coherent power spectrum.

9. Pulse measuring device (1) according to claim 8, **characterised in that** the microcontroller unit (3) comprises memory means (11, 12) in which there are stored instructions for setting the microcontroller unit in operation, configuration parameters which are specific to said unit, data relating to the digitalised measurement signals provided by the photodetector and processed by the processing unit, and **in that** the processing unit (9) is able to calculate at least one coherent power spectrum on the basis of a spectral coherence function $\gamma$ in order to determine the frequency of the movement of the person by means of the coherent power spectrum.

10. Device for measuring the pulse (1) according to claim 8, **characterised in that** it comprises a display cell (4) controlled by the microcontroller unit (3), said display cell being able to display information relating to the heartbeat and/or to the movement of the person carrying said device.

11. Device for measuring the pulse (1) according to claim 8, **characterised in that** the microcontroller unit (3) comprises an analogue-digital converter (8) for digitally converting the measurement signals provided by the photodetector (7) in order to be processed by the processing unit (9), and at least one digital-analogue converter (10) for controlling alternately, on the basis of control signals from the processing unit, the activation of each light source (5, 6), each light source being an electroluminescent diode.

12. Portable electronic instrument, such as a wristwatch, which comprises a pulse measuring device (1) according to one of the claims 8 to 11, **characterised in that** the first and second light sources (5, 6) are disposed in a case of the instrument for providing the light waves through a base of the case, and **in that** the photodetector (7) is provided in order to sense the light waves reflected through an opening or transparent portion of the base of the case.

Fig. 1

Dispositif de mesure du pouls

Affichage

Unité à microcontrôleur

Bus de données et commandes

Ext. I/F

CPU
ADC
DACs
RAM
ROM
Batterie

Unité de détection optique

LED1
LED2
Photo-détecteur

épiderme

derme, vaisseaux sanguins

$\lambda_1$ $\lambda_2$

1  4  30  3  11  12  13  2  5  10  8  9  7  6

Fig. 2

EP 2 292 141 B1

**Signaux temporels verts et rouges en présence de mouvement**

Axe Y : Signaux (unités arbitraires) — graduations 1.00, 0.50, 0.00, -0.50, -1.00

Axe X : Temps en secondes — graduations 50, 52, 54, 56, 58, 60

Légende :
— Vert
- - - Rouge

Fig. 3

EP 2 292 141 B1

Fig. 4

Fig. 5

Fig. 6

EP 2 292 141 B1

Impulsions lumineuses LED1

$T_{cycle}$

$T_{pulse}$

$T_{int}$

Impulsions lumineuses LED2

$T_{pulse}$

$T_{dm}$

$T_{mes}$

Mesures: Photo-détecteur et convertisseur A/N

Mesure LED1

Mesure lumière ambiante

Mesure LED2

Fig. 7

EP 2 292 141 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4770179 A
- FR 2511594
- US 20050075553 A
- EP 0941694 A